# EUROPÄISCHE PATENTANMELDUNG

(11) **EP 1 982 977 A1**
(43) Veröffentlichungstag der Anmeldung: **22.10.2008**
(21) Anmeldenummer: 07008087.4
(22) Anmeldetag: 20.04.2007
(51) Int. Cl.: C07D 213/06, C07D 213/79, C07D 213/81

(54) **Verfahren zur Hydrolyse von heteroaromatischen Nitrilen in wässrigen Fluiden**

(71) Anmelder: Lonza AG, 4052 Basel (CH)
(72) Erfinder: Die Erfindernennung liegt noch nicht vor

(57) **Zusammenfassung**

Die vorliegende Erfindung betrifft ein Verfahren zur Hydrolyse von Verbindungen der Formeln 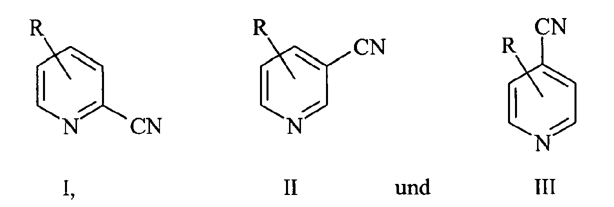 worin R Wasserstoff oder C₁₋₂₀-Alkyl ist, zu den entsprechenden Amiden und/oder Säuren in wässrigen Fluiden.

## Beschreibung

Die vorliegende Erfindung betrifft ein Verfahren zur Hydrolyse von Verbindungen der Formeln 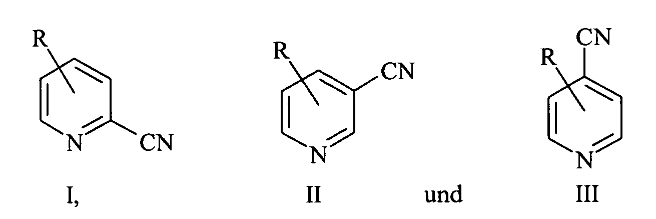 worin R Wasserstoff oder C₁₋₂₀-Alkyl ist, in wässrigen Fluiden unter Hochdruckbedingungen.

Heteroaromatische Nitrile der Formeln I oder II, insbesondere Nikotinsäurenitril (NSN), sind wichtige Intermediate unter anderem zur Herstellung der sogenannten Nikotinate, wie Nikotinsäureamid (NSA) und Nikotinsäure (NS). NSA und NS werden beispielsweise Nahrungsmitteln als Vitamine zugesetzt oder als Bausteine für die Herstellung pharmazeutisch wirksamer Verbindungen verwendet.

Es ist bekannt, dass Verbindungen der Formeln I bis III schrittweise über die Säureamide der Formeln 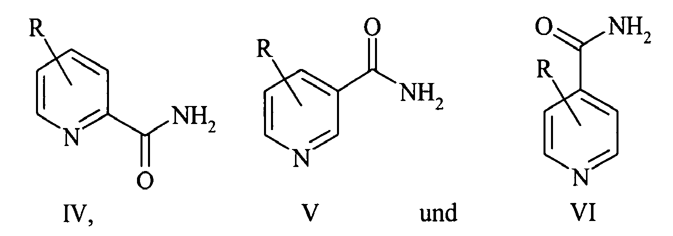 worin R wie oben definiert ist, zu den Säuren der Formel 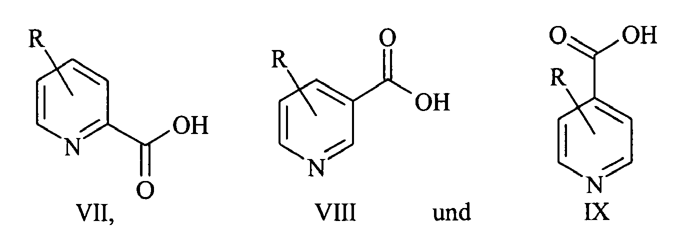 worin R wie oben definiert ist, hydrolysiert werden können.

In den bekannten Verfahren zur Herstellung von NSA und NS aus NSN entstehen jedoch hohe Salzfrachten im Produktionsablauf und stossen daher zunehmend auf Akzeptanzprobleme. Seit einiger Zeit wachsen die Anforderungen an die Reduzierung der Salzfracht in Produktionsabläufen, da sich die Beseitigung solcher Abwässer, beispielsweise durch Verbrennung oder Verklappung zunehmend verteuert. Weiterhin lassen sich die Salze nur aufwendig aus den teilweise ebenfalls in Salzform vorliegenden Produkten entfernen. Diese Salzfracht ist insbesondere bei NSA und NS problematisch wenn diese als Ernährungs- oder Futtermittelzusätze verwendet werden.

Gemäss US 5756750 kann die Hydrolyse von NSN bei niedriger Natronlaugekonzentration (14 Teile Base zu 100 Teilen NSN) zu NSA und bei äquimolarer Natronlaugekonzentration zu NS ablaufen. Nach Neutralisation mit HCl fällt etwa ein Teil NaCl pro 2 Teile NS als Abfallprodukt an.
Eine weitere Möglichkeit zur Darstellung von NS aus NSN ist die enzymatische Hydrolyse. Die dabei eingesetzten Nitrilasen benötigen meist komplexe Medienzusammensetzungen. Weiterhin liefern diese Enzyme geringe Raum-Zeit-Ausbeuten und erfordern ein Aussalzen der NS mit daraus resultierendem Salzanfall *(*Process Biochemistry 2006, 41(9), 2078-2081 und JP 2005176639). Im enzymatischen Verfahren ist weiterhin oftmals ein Aktivitätsverlust der Enzyme festzustellen (Journal of Molecular Catalysis B: Enzymatic 2006, 39(1-4), 55-58).

Aufgabe der vorliegenden Erfindung war daher ein Verfahren zu entwickeln, das sich durch eine deutliche Reduzierung der Salzfrachten bei hohen Raum-Zeit-Ausbeuten auszeichnet.

Diese Aufgabe wurde entsprechend Anspruch 1 gelöst.

Beansprucht wird ein Verfahren zur Hydrolyse von Verbindungen der Formeln 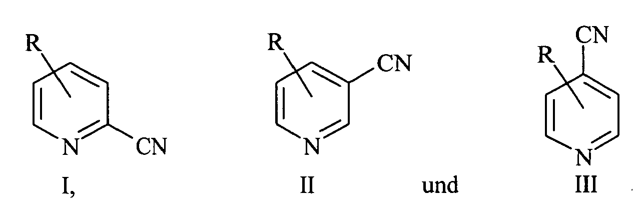 worin R Wasserstoff oder C₁₋₂₀-Alkyl ist,
zu den entsprechenden Amiden und/oder Säuren in wässrigen Fluiden, bei einer Temperatur von mindestens 100 °C, vorzugsweise mindestens 150 °C, besonders bevorzugt von mindestens 200 °C, und einem Druck von mindestens 20 kPa, gegebenenfalls in Gegenwart eines sauren oder basischen Katalysators.

Je nach Reaktionsführung werden Gemische unterschiedlicher Zusammensetzung erhalten. Die erste Stufe der Hydrolyse des Ausgangsmaterials erfolgt unter den erfindungsgemässen Bedingungen so schnell, dass je nach geeigneter Reaktionskontrolle fast ausschliesslich die Amide der Formeln IV bis VI oder die entsprechenden Säuren der Formeln VII bis IX erhalten werden können. In den unten angeführten Beispielen wird gezeigt, dass die Produktzusammensetzung durch Wahl geeigneter Parameter (Temperatur, Druck, Katalysatorart und -menge) über einen grossen Bereich gesteuert werden kann. Dies ist insbesondere von Vorteil für die Verbindungen NSA und NS, welche im Ernährungs- und Futtermittelbereich auch als Gemische verwendet werden können, und bei denen das Produkt - nach Abtrennung der gegebenenfalls verwendeten Lösungsmittel und Katalysatoren - direkt weiterverwendet werden kann.

Zweckmässig wird im erfindungsgemässen Verfahren für die bevorzugte Herstellung von Verbindungen der Formeln IV bis IX eine Temperatur von 400 °C nicht überschritten. Oberhalb 400 °C findet fast quantitativ die Decarboxylierung der Säuren der Formeln VII bis IX zu den entsprechenden Pyridinderivaten statt.

In einer bevorzugten Verfahrensvariante wird die Reaktion kontinuierlich in einem Strömungsreaktor durchgeführt.

Geeignete Reaktoren sind beispielsweise Strömungsreaktoren mit oder ohne statische Mischer oder druckfeste Mikroreaktoren mit oder separate Mischzonen. Bei der Zuführung von homogenen Gemischen kann auf besondere Mischeinrichtungen verzichtet werden. Bei getrennter Zuführung von Edukten und wässriger Phase ist eine Mischeinrichtung vorteilhaft. Unter den Mikroreaktoren auf sind insbesondere solche auf Metallbasis geeignet, die sich schnell und genau temperieren lassen. Gegebenenfalls enthalten geeignete Strömungsreaktoren zusätzliche Zuführungen für die Einspeisung von Katalysatoren entlang der Reaktionszone. Diese zusätzliche Einspeisung ist insbesondere für die Einleitung von gasförmigem Ammoniak bei basischer Reaktionsführung vorteilhaft.

Geeignete Strömungsreaktoren weisen mindestens eine Reaktionszone und gegebenenfalls eine oder mehrere Nachreaktionszone auf.

In einem statischen Mischer beträgt die Verweildauer in der Reaktionszone vorzugsweise zwischen 0,1 und 2000 Sekunden, besonders bevorzugt zwischen 30 und 1000 Sekunden.

Unter wässrigen Fluiden werden im vorliegenden Verfahren Wasser enthaltende Lösungsmittel, insbesondere wässrige Flüssigkeiten, verstanden, in welchen die übrigen Lösungsmittelbestandteile inert gegen über den Ausgangsverbindungen, den Produkten sind und sich unter den Reaktionsbedingungen nicht zersetzen. Als Lösungsmittelbestandteile kommen neben Wasser auch organische Verbindungen wie C₁₋₁₀-Alkohole, C₁₋₁₀-Carbonsäuren, Ester der vorgenannten Alkoholen und Carbonsäuren, Methyl-*tert*-butylketon und Paraffine in Betracht. Die genannten Carbonsäuren können hierbei, insbesondere bei den hohen Reaktionstemperaturen, gleichzeitig als Lösungsmittel und als Katalysator wirken. Vorzugsweise besteht das Fluid überwiegend aus Wasser.

In einer bevorzugten Verfahrensvariante enthält das wässrige Fluid zu Beginn der Reaktion die Verbindung der Formel I in einer Menge von 0,05 bis 30 Gew.-%, besonders bevorzugt von 10 bis 20 Gew.-%

In einer weiteren bevorzugten Verfahrensvariante wird ein Druck von 20 bis 60 kPa verwendet, besonders bevorzugt zwischen 20 und 35 kPa.

Generell überwiegt mit steigender Temperatur bzw. mit steigender Verweilzeit im Reaktor die Bildung der Säuren der Formeln VII bis IX gegenüber der Bildung der Amide der Formeln IV bis VI. Wird das Verfahren in einem wässrigen Fluid bei nicht vorgegebenem pH durchgeführt - also ohne Zusatz von Säuren oder Basen -, lässt sich das Produktverhältnis der Säuren zu den Amiden durch Temperatur und Druck in einem weiten Bereich steuern. Die Reaktionszeiten unter diesen Bedingungen sind jedoch relativ lange und begünstigen die Decarboxylierung der gebildeten Nikotinsäure zu den entsprechenden Pyridinderivaten.

Zur Erhöhung der Reaktionsgeschwindigkeit und zur weiteren Steuerung der Selektivität können im erfindungsgemässen Verfahren vorteilhaft auch basische oder saure Katalysatoren zugesetzt werden. Basische und saure Katalysatoren können auch die Bildung von Nebenprodukten reduzieren. Als Katalysatoren eigenen sich sowohl im Reaktionsgemisch wenigstens teilweise lösliche Säuren und Basen wie auch heterogene Säuren und Basen.

Bei Verwendung von sauren Katalysatoren wird neben einer allgemeinen Steigerung der Reaktionsgeschwindigkeit die Bildung unerwünschter decarboxylierter Pyridine begünstigt.

Bei Verwendung von basischen Katalysatoren wird neben einer allgemeinen Steigerung der Reaktionsgeschwindigkeit die Selektivität hinsichtlich der Bildung der Säuren der Formeln VII bis IX verbessert. Bei vergleichbaren Temperaturen und Reaktionszeiten hat die Verwendung basischer Katalysatoren gegenüber dem Verfahren unter Verwendung saurer Katalysatoren oder in Abwesenheit von Katalysatoren den weiteren Vorteil einer reduzierten Decarboxylierung.

In einer besonders bevorzugten Verfahrensvariante ist der Katalysator ein saurer Katalysator.

Als saure Katalysatoren eignen sich insbesondere niedermolekulare organische Säuren, wie beispielsweise lineare oder verzweigte C₁₋₆-Carbonsäuren, oder anorganische Säuren, wie beispielsweise oxidierende oder nichtoxidierende Protonensäuren oder Lewis-Säuren, aber auch anorganische Festkörpersäuren wie Poly- und Heteropolysäuren und Zeolithe.

Die anorganischen Säuren werden vorzugsweise ausgewählt aus der Gruppe bestehend aus Halogenwasserstoffsäuren, Schwefelsäure, Schweflige Säure, Phosphorsäure, Phosphorige Säure, Lewis-Säuren, Polyphosphor- und Polywolframsäuren, saure Zeolithe, saure Tone und Mischungen davon.

In einer alternativen Verfahrensvariante ist der Katalysator ein basischer Katalysator, insbesondere eine organische oder anorganische Base.

Als basischer Katalysator eignet sich insbesondere eine Base, ausgewählt aus der Gruppe bestehend aus Hydroxiden und Oxiden von Metallen der ersten und zweiten Hauptgruppe, Ammoniak, Triethylamin, Trimethylamin und Mischungen davon. Insbesondere geeignet sind Alkali- und Erdalkalimetallhydroxide, Alkalimetalloxide und Ammoniak.

Eine katalytische Wirkung tritt im Falle von basischen Katalysatoren bereits in Konzentrationen ab 100 ppm bezogen auf das Ausgangsprodukt auf. In einer bevorzugten Ausführungsform bei basischer Katalyse ist der pH > 7. Basische Katalysatoren können in beliebiger Menge verwendet werden.

Da die Reaktion unter Druck stattfindet, besteht die Möglichkeit gasförmige Basen wie Ammoniak oder Trimethylamin unter Druck zuzusetzen. Besonders bevorzugt wird Ammoniak als gasförmige Base verwendet. In einer ganz besonders bevorzugten Ausführungsform wird in Gegenwart eines basischen Katalysators zusätzlich gasförmiges Ammoniak unter Druck zugegeben. Ammoniak als basischer Katalysator ist besonders vorteilhaft, da Ammoniak beispielsweise im Vakuum bequem aus dem Produkt entfernt werden kann und somit keine Neutralisation erfordert.

### Beispiele:

Die folgenden Beispiele verdeutlichen die Ausführung der Erfindung, ohne dass darin eine Einschränkung zu sehen ist.

In den Ergebnistabellen der Beispiele 1-4 werden in den einzelnen Spalten die Werte für die Reaktionsdauer τ in [s], für die Konzentration des Nitrils zu verschiedenen Reaktionszeiten c_{CN} in [mM], für die Konzentration der gebildeten Säure zu verschiedenen Reaktionszeiten c_{COOH} in [mM], für die Konzentration des gebildeten Amids zu verschiedenen Reaktionszeiten c_{CONH2} in [mM], für den Umsatz des Nitrils U_{CN} in [%], Für den Flächenanteil der gebildeten Säure A_{CO0H} in [%], für den Flächenanteil des gebildeten Amids A_{CONH2} in %, für die Selektivität der gebildeten Säure S_{COOH} in [%] sowie für die Selektivität des gebildeten Amids A_{CONH2} in [%] angegeben.
n.b. = nicht bestimmt

### Beispiel 1: (Tabellen 1 bis 7) Hydrolyse von 0,5 Gew.-% Nikotinonitril in reinem Wasser

### Beispiel 2: (Tabellen 8 bis 18) Hydrolyse von 0,5 Gew.-% Nikotinonitril in 10 mmol Schwefelsäure

### Beispiel 3: (Tabellen 19 bis 42) Hydrolyse von 0,5 Gew.-% Nikotinonitril in verschieden konzentrierten Ammoniaklösungen

### Beispiel 4: (Tabellen 43 bis 53) Hydrolyse von 5, 10 und 15 Gew.-% Nikotinonitril in konz. Ammoniak (14,7 M, 25%)

### Beispiel 1: Hydrolyse von 0,5 % Nikoktinonitril in reinem Wasser

**Tabelle 1: Messdaten und Ergebnisse bei 200 °C und 25 MPa in Wasser**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 96,9 | 0 | 0 | 0 | 0 | 0 | | |
| 50 | 94,6 | 0 | 0,85 | 2,3 | 0 | 0,9 | 0 | 38 |
| 72 | 93,6 | 0,06 | 1,33 | 3,4 | 0,1 | 1,4 | 1,8 | 40,5 |
| 101 | 92,3 | 0,13 | 1,82 | 4,7 | 0,1 | 1,9 | 2,9 | 40 |
| 206 | 87,3 | 0,14 | 3,59 | 9,9 | 0,1 | 3,7 | 1,5 | 38 |
| 269 | 84,2 | 0,15 | 4,74 | 13,0 | 0,2 | 4,9 | 1,2 | 37,5 |

**Tabelle 2: Messdaten und Ergebnisse bei 250 °C und 25 MPa in Wasser**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 83,9 | 0,12 | 0 | 0 | 0 | 0 | | |
| 59 | 76,9 | 0,32 | 5,57 | 8,3 | 0,2 | 6,6 | 2,88 | 80,4 |
| 74 | 75,3 | 0,35 | 7,30 | 10,2 | 0,3 | 8,7 | 2,73 | 85,6 |
| 101 | 73,6 | 0,41 | 8,80 | 12,2 | 0,3 | 10,5 | 2,81 | 85,9 |
| 195 | 68,3 | 0,65 | 12,68 | 18,6 | 0,6 | 15,1 | 3,37 | 81,3 |
| 325 | 63,6 | 0,84 | 15,59 | 24,2 | 0,9 | 18,6 | 3,54 | 76,8 |
| 390 | 61,2 | 0,97 | 16,76 | 27,0 | 1,0 | 20,0 | 3,75 | 74,1 |

**Tabelle 3: Messdaten und Ergebnisse bei 300 °C und 25 MPa in Wasser**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 75,6 | 0,11 | 0 | 0 | 0 | 0 | | |
| 37 | 67,2 | 0,31 | 7,60 | 11,1 | 0,3 | 10,0 | 2,31 | 90,6 |
| 50 | 64,6 | 0,39 | 10,61 | 14,6 | 0,4 | 14,0 | 2,50 | 96,4 |
| 78 | 60,9 | 0,51 | 13,96 | 19,4 | 0,5 | 18,5 | 2,68 | 95,2 |
| 100 | 58,7 | 0,64 | 16,02 | 22,3 | 0,7 | 21,2 | 3,13 | 94,9 |
| 191 | 50 | 1,49 | 23,05 | 33,8 | 1,8 | 30,5 | 5,38 | 90,3 |
| 300 | 41,7 | 3,41 | 29,02 | 44,8 | 4,4 | 38,4 | 9,75 | 85,7 |
| 377 | 36,1 | 5,17 | 32,20 | 52,3 | 6,7 | 42,6 | 12,80 | 81,5 |

**Tabelle 4: Messdaten und Ergebnisse bei 350 °C und 25 MPa in Wasser**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 62,18 | 0 | 0 | 0 | 0 | 0 | | |
| 47,5 | 45,32 | 1,53 | 14,60 | 40,1 | 2,5 | 23,5 | 9,09 | 86,6 |
| 99 | 36,99 | 3,33 | 20,75 | 51,1 | 5,4 | 33,4 | 13,22 | 82,4 |
| 218 | 26,61 | 6,01 | 27,43 | 64,8 | 9,7 | 44,1 | 16,91 | 77,1 |
| 288 | 20,66 | 7,61 | 31,11 | 72,7 | 12,2 | 50,0 | 18,34 | 75,0 |
| 389 | 13,04 | 9,45 | 35,30 | 82,7 | 15,2 | 56,8 | 19,24 | 71,8 |
| 496 | 6,54 | 11,14 | 37,87 | 91,4 | 17,9 | 60,9 | 20,03 | 68,1 |

**Tabelle 5: Messdaten und Ergebnisse bei 380 °C und 25 MPa in Wasser**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 55,1 | 0 | 0 | 0 | 0 | 0 | | |
| 47 | 36,1 | 0,67 | 10,31 | 34,4 | 1,2 | 18,7 | 3,55 | 54,3 |
| 202 | 28,9 | 0,38 | 13,37 | 47,6 | 0,7 | 24,2 | 1,47 | 51,0 |
| 293 | 28 | 0,07 | 13,88 | 49,2 | 0,1 | 25,2 | 0,27 | 51,2 |
| 392 | 27 | 0 | 14,29 | 51,0 | 0 | 25,9 | 0 | 50,8 |
| 486 | 27,1 | 0 | 13,86 | 50,7 | 0 | 25,1 | 0 | 49,5 |

**Tabelle 6: Messdaten und Ergebnisse bei 400 °C und 25 MPa in Wasser**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 19,52 | 0 | 0 | 0 | 0 | 0 | | |
| 10 | 16,35 | 0,42 | 2,41 | 16,3 | 2,2 | 12,3 | 13,32 | 75,9 |
| 25 | 12,84 | 0,93 | 4,38 | 34,2 | 4,7 | 22,4 | 13,86 | 65,5 |
| 50 | 8,77 | 1,02 | 5,86 | 55,1 | 5,2 | 30,0 | 9,45 | 54,5 |
| 130 | 4,38 | 0,30 | 6,39 | 77,6 | 1,6 | 32,7 | 2,01 | 42,2 |
| 203 | 3,20 | 0,08 | 6,07 | 83,6 | 0,4 | 31,1 | 0,51 | 37,2 |

**Tabelle 7: Messdaten und Ergebnisse bei 450 °C und 25 MPa in Wasser**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 13 | 0 | 0 | 0 | 0 | 0 | | |
| 9,7 | 9,02 | 0,89 | 2,66 | 31 | 6,9 | 20,5 | 22,45 | 66,8 |
| 25,2 | 3,13 | 1,07 | 3,19 | 76 | 8,3 | 24,6 | 10,87 | 32,3 |
| 49,4 | 1,00 | 0,50 | 2,20 | 92 | 3,8 | 16,9 | 4,15 | 18,4 |
| 126,8 | 0 | 0 | 0,08 | 100 | 0,0 | 0,6 | 0 | 0,6 |

### Beispiel 2: Hydrolyse von und 0,5 Gew.-% Nikotinonitril mit Schwefelsäure

**Tabelle 8: Messdaten und Ergebnisse bei 250 °C und 25 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 50,5 | 0 | 0 | 0 | 0 | 0 | | |
| 45 | 48,7 | 0,27 | 0,87 | 3,4 | 0,5 | 1,7 | 15,50 | 50,0 |
| 72 | 47,7 | 0,87 | 1,37 | 5,5 | 1,7 | 2,7 | 31,66 | 49,5 |
| 98 | 46,9 | 1,34 | 1,73 | 7,0 | 2,7 | 3,4 | 37,89 | 48,9 |
| 180 | 44,2 | 2,76 | 2,93 | 12,5 | 5,5 | 5,8 | 43,69 | 46,5 |
| 255 | 41,8 | 3,90 | 3,93 | 17,2 | 7,7 | 7,8 | 44,95 | 45,4 |
| 379 | 39,3 | 4,71 | 4,87 | 22,2 | 9,3 | 9,6 | 42,02 | 43,5 |

**Tabelle 9: Messdaten und Ergebnisse bei 300 °C und 25 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 39,8 | 0 | 0 | 0 | 0 | 0 | | |
| 47 | 34 | 2,68 | 2,96 | 14,7 | 6,7 | 7,4 | 45,74 | 50,5 |
| 76 | 30,7 | 3,95 | 4,41 | 22,9 | 9,9 | 11,1 | 43,29 | 48,2 |
| 100 | 28,5 | 4,68 | 5,31 | 28,5 | 11,8 | 13,3 | 41,28 | 46,8 |
| 193 | 23,3 | 5,96 | 7,12 | 41,4 | 15,0 | 17,9 | 36,16 | 43,2 |
| 280 | 20,3 | 6,63 | 8,03 | 49,1 | 16,7 | 20,2 | 33,91 | 41,0 |
| 384 | 17,5 | 7,18 | 8,75 | 56,1 | 18,0 | 22,0 | 32,14 | 39,2 |

**Tabelle 10: Messdaten und Ergebnisse bei 330 °C und 25 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 32,95 | 0 | 0 | 0 | 0 | 0 | | |
| 49 | 25,04 | 2,90 | 2,88 | 24 | 8,8 | 8,7 | 36,63 | 36,3 |
| 73 | 22,69 | 2,45 | 3,87 | 31 | 7,4 | 11,7 | 23,89 | 37,7 |
| 94 | 21,05 | 2,12 | 4,53 | 36 | 6,4 | 13,8 | 17,80 | 38,1 |
| 190 | 16,35 | 1,56 | 6,08 | 50 | 4,7 | 18,5 | 9,40 | 36,6 |
| 301 | 12,42 | 1,23 | 7,02 | 62 | 3,7 | 21,3 | 5,97 | 34,2 |
| 377 | 10,46 | 1,00 | 7,35 | 68 | 3 | 22,3 | 4,46 | 32,7 |

**Tabelle 11: Messdaten und Ergebnisse bei 350 °C und 25 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 28,42 | 0 | 0 | 0 | 0 | 0 | | |
| 62 | 21,03 | 1,08 | 3,05 | 47,2 | 3,8 | 10,7 | 14,55 | 41,3 |
| 85 | 18,62 | 0,82 | 3,61 | 53,2 | 2,9 | 12,7 | 8,37 | 36,8 |
| 101 | 16,82 | 0,67 | 3,96 | 57,8 | 2,3 | 13,9 | 5,74 | 34,2 |
| 174 | 12,50 | 0,51 | 4,57 | 68,6 | 1,8 | 16,1 | 3,22 | 28,7 |
| 308 | 8,29 | 0,41 | 4,67 | 79,2 | 1,4 | 16,4 | 2,04 | 23,2 |
| 384 | 6,67 | 0,41 | 4,37 | 83,3 | 1,4 | 15,4 | 1,88 | 20,1 |

**Tabelle 12: Messdaten und Ergebnisse bei 380 °C und 25 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 22,21 | 0 | 0 | 0 | 0 | 0 | | |
| 33,6 | 14,29 | 0,63 | 2,93 | 35,7 | 2,8 | 13,2 | 7,92 | 37,0 |
| 52,1 | 11,09 | 0,55 | 3,41 | 50,1 | 2,5 | 15,3 | 4,98 | 30,6 |
| 88,8 | 7,79 | 0,48 | 3,63 | 64,9 | 2,2 | 16,3 | 3,33 | 25,1 |
| 202,5 | 4,11 | 0,33 | 2,93 | 81,5 | 1,5 | 13,2 | 1,84 | 16,2 |
| 286,2 | 2,30 | 0,15 | 2,05 | 89,7 | 0,7 | 9,2 | 0,74 | 10,3 |
| 434,0 | 0 | 0 | 0,11 | 100,0 | 0,0 | 0,5 | 0,00 | 0,5 |

**Tabelle 13: Messdaten und Ergebnisse bei 400 °C und 25 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 8,08 | 0 | 0,63 | 0 | 0 | 0 | | |
| 14,2 | 2,78 | 0,63 | 0,35 | 65,5 | 7,8 | -3,4 | 11,85 | -5,2 |
| 25,6 | 1,54 | 0,35 | 0,14 | 81,0 | 4,4 | -6,1 | 5,42 | -7,5 |
| 45,6 | 0,75 | 0,14 | 0,03 | 90,7 | 1,7 | -7,4 | 1,86 | -8,2 |
| 83,9 | 0,24 | 0,03 | 0,00 | 97,0 | 0,3 | -7,8 | 0,35 | -8,0 |
| 179,9 | 0,03 | 0,00 | 0,00 | 99,6 | 0 | -7,8 | 0 | -7,8 |

| | | | | | | | | |
|---|---|---|---|---|---|---|---|---|
| Bei 400 °C zeigten sich deutliche Zersetzungserscheinungen. | | | | | | | | |

**Tabelle 14: Messdaten und Ergebnisse bei 250 °C und 30 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 41,1 | 0 | 0 | 0 | 0 | 0 | | |
| 47 | 39,6 | 0,3 | 0,9 | 3,7 | 0,7 | 2,1 | 17,8 | 58,3 |
| 75 | 38,5 | 0,8 | 1,4 | 6,2 | 2,0 | 3,5 | 32,0 | 55,9 |
| 100 | 37,3 | 1,4 | 2,0 | 9,1 | 3,5 | 4,9 | 37,9 | 54,1 |
| 182 | 34,6 | 3,2 | 3,2 | 15,9 | 7,7 | 7,9 | 48,4 | 49,8 |
| 278 | 32,3 | 4,6 | 4,1 | 21,4 | 11,3 | 10,0 | 52,6 | 46,8 |
| 385 | 30,1 | 5,9 | 5,0 | 26,6 | 14,4 | 12,2 | 53,9 | 45,7 |
| 883 | 22,0 | 10,4 | 8,1 | 46,5 | 25,3 | 19,7 | 54,4 | 42,4 |

**Tabelle 15: Messdaten und Ergebnisse bei 300 °C und 30 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 40,4 | 0 | 0 | 0 | 0 | 0 | | |
| 59 | 33,5 | 3,4 | 3,1 | 17,0 | 8,5 | 7,6 | 49,8 | 44,9 |
| 101 | 30,5 | 4,6 | 4,2 | 24,5 | 11,3 | 10,5 | 46,3 | 42,9 |
| 190 | 24,3 | 6,4 | 6,6 | 39,8 | 15,9 | 16,3 | 39,8 | 40,9 |
| 363 | 16,6 | 7,1 | 8,9 | 58,9 | 17,7 | 22,1 | 30,0 | 37,5 |
| 558 | 11,2 | 7,0 | 10,3 | 72,3 | 17,4 | 25,6 | 24,0 | 35,4 |

**Tabelle 16: Messdaten und Ergebnisse bei 330 °C und 30 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 41,3 | 0 | 0 | 0 | 0 | 0 | | |
| 47 | 33,4 | 3,1 | 3,6 | 19,0 | 7,5 | 8,8 | 39,4 | 46,2 |
| 74 | 30,3 | 3,7 | 4,7 | 26,6 | 8,9 | 11,4 | 33,3 | 42,9 |
| 99 | 26,6 | 4,2 | 5,9 | 35,5 | 10,1 | 14,3 | 28,4 | 40,3 |
| 182 | 18,6 | 3,9 | 8,2 | 54,8 | 9,5 | 19,9 | 17,4 | 36,4 |
| 270 | 12,0 | 3,0 | 9,6 | 71,0 | 7,2 | 23,4 | 10,2 | 32,9 |
| 376 | 6,0 | 2,0 | 10,5 | 85,5 | 4,8 | 25,4 | 5,6 | 29,8 |
| 471 | 2,7 | 1,7 | 10,8 | 93,5 | 4,1 | 26,1 | 4,4 | 27,9 |

**Tabelle 17: Messdaten und Ergebnisse bei 350 °C und 30 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 38,3 | 0 | 0 | 0 | 0 | 0 | | |
| 48 | 26,2 | 1,7 | 4,3 | 35,2 | 4,5 | 11,3 | 14,2 | 35,5 |
| 95 | 16,7 | 1,7 | 6,3 | 58,6 | 4,5 | 16,5 | 8,0 | 29,2 |
| 186 | 10,2 | 0,6 | 6,3 | 74,7 | 1,6 | 16,5 | 2,2 | 22,5 |
| 267 | 6,8 | 0,5 | 5,7 | 83,1 | 1,2 | 14,8 | 1,5 | 18,1 |
| 379 | 3,7 | 0,3 | 3,7 | 90,8 | 0,8 | 9,8 | 0,9 | 10,8 |

**Tabelle 18: Messdaten und Ergebnisse bei 380 °C und 30 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 32,6 | 0 | 0 | 0 | 0 | 0 | | |
| 49 | 16,5 | 0,3 | 4,3 | 49,4 | 0,9 | 13,3 | 1,9 | 26,9 |
| 97 | 7,8 | 0,2 | 3,8 | 75,9 | 0,7 | 11,8 | 0,9 | 15,6 |
| 190 | 1,3 | 0,1 | 1,9 | 95,9 | 0,3 | 5,9 | 0,3 | 6,2 |
| 288 | 0,1 | 0,0 | 0,8 | 99,7 | 0,1 | 2,6 | 0,1 | 2,6 |
| 375 | 0,0 | 0,0 | 0,3 | 100,0 | 0,0 | 0,9 | 0 | 0,9 |

### Beispiel 3: Hydrolyse von und 0,5 Gew.-% Nikotinonitril in verschieden konzentrierten Ammoniaklösungen

**Tabelle 19: Messdaten und Ergebnisse bei 250 °C und 30 MPa in 5 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 43,93 | 0 | 0,00 | 0 | 0 | 0 | | |
| 56,2 | 22,04 | 1,08 | 19,92 | 49,8 | 2,5 | 45,3 | 4,94 | 91,0 |
| 97,6 | 18,24 | 2,03 | 22,93 | 58,5 | 4,6 | 52,2 | 7,89 | 89,3 |
| 182,6 | 12,29 | 3,92 | 26,96 | 72,0 | 8,9 | 61,4 | 12,39 | 85,2 |
| 286,3 | 8,01 | 6,08 | 29,10 | 81,8 | 13,8 | 66,2 | 16,94 | 81,0 |
| 421,3 | 4,76 | 9,13 | 29,04 | 89,2 | 20,8 | 66,1 | 23,30 | 74,1 |
| 549,5 | 2,85 | 11,69 | 28,03 | 93,5 | 26,6 | 63,8 | 28,47 | 68,2 |
| 712,1 | 1,35 | 15,61 | 26,15 | 96,9 | 35,5 | 59,5 | 36,67 | 61,4 |

**Tabelle 20: Messdaten und Ergebnisse bei 250 °C und 30 MPa in 50 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 39,33 | 0 | 0 | 0 | 0 | 0 | | |
| 49 | 8,01 | 1,55 | 28,16 | 79,6 | 4,0 | 71,6 | 4,96 | 89,9 |
| 74 | 6,42 | 2,37 | 28,84 | 83,7 | 6,0 | 73,3 | 7,19 | 87,6 |
| 102,3 | 5,15 | 3,38 | 29,17 | 86,9 | 8,6 | 74,2 | 9,89 | 85,4 |
| 190,6 | 2,70 | 6,56 | 29,00 | 93,1 | 16,7 | 73,7 | 17,90 | 79,2 |
| 296,1 | 1,43 | 9,67 | 27,56 | 96,4 | 24,6 | 70,1 | 25,50 | 72,7 |
| 379,5 | 0,87 | 12,17 | 26,29 | 97,8 | 30,9 | 66,8 | 31,64 | 68,4 |
| 539,3 | 0,16 | 16,09 | 22,93 | 99,6 | 40,9 | 58,3 | 41,07 | 58,5 |

**Tabelle 21: Messdaten und Ergebnisse bei 250 °C und 30 MPa in 1,17 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 35,30 | 0 | 0,57 | 0 | 0 | 0 | | |
| 45,9 | 0,25 | 4,73 | 30,25 | 99,3 | 13,4 | 84,1 | 13,51 | 84,7 |
| 73,4 | 0 | 7,40 | 27,21 | 100,0 | 21,0 | 75,5 | 20,95 | 75,5 |
| 99,8 | 0 | 9,77 | 25,08 | 100,0 | 27,7 | 69,4 | 27,67 | 69,4 |
| 171,9 | 0 | 14,71 | 20,53 | 100,0 | 41,7 | 56,6 | 41,68 | 56,6 |
| 292,0 | 0 | 18,46 | 16,58 | 100,0 | 52,3 | 45,4 | 52,29 | 45,4 |
| 446,9 | 0 | 21,34 | 13,28 | 100,0 | 60,5 | 36,0 | 60,46 | 36,0 |

**Tabelle 22: Messdaten und Ergebnisse bei 250 °C und 30 MPa in 5,88 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 38,30 | 0 | 0,23 | 0 | 0 | 0 | | |
| 44,8 | 0 | 10,40 | 25,22 | 100,0 | 27,2 | 65,2 | 27,15 | 65,2 |
| 74,7 | 0 | 17,17 | 21,08 | 100,0 | 44,8 | 54,4 | 44,81 | 54,4 |
| 104,4 | 0 | 21,11 | 17,31 | 100,0 | 55,1 | 44,6 | 55,12 | 44,6 |
| 188,4 | 0 | 26,89 | 11,54 | 100,0 | 70,2 | 29,5 | 70,21 | 29,5 |
| 291,3 | 0 | 30,59 | 7,48 | 100,0 | 79,9 | 18,9 | 79,87 | 18,9 |
| 464,7 | 0 | 32,67 | 4,05 | 100,0 | 85,3 | 10,0 | 85,28 | 10,0 |
| 679,5 | 0 | 33,76 | 1,74 | 100,0 | 88,1 | 3,9 | 88,13 | 3,9 |

**Tabelle 23: Messdaten und Ergebnisse bei 250 °C und 30 MPa in 14,7 M Ammoniak**

| τ | c_{CN} | c_{COOH} | c_{CONH2} | U_{CN} | A_{COOH} | A_{CONH2} | S_{COOH} | ^{S}CONH2 |
|---|---|---|---|---|---|---|---|---|
| 0 | 37,03 | 0 | 3,15 | 0 | 0 | 0 | | |
| 43,9 | 0 | 8,86 | 30,18 | 100 | 22,0 | 73, | 23,91 | 73 |
| 118,7 | 0 | 21,36 | 18,31 | 100 | 53,2 | 40,9 | 57,68 | 40,9 |
| 225,5 | 0 | 29,54 | 9,59 | 100 | 73,5 | 17,4 | 79,77 | 17,4 |
| 343,3 | 0 | 33,6 | 5,50 | 100 | 83,6 | 6,3 | 90,72 | 6,3 |
| 561,8 | 0 | 36,1 | 3,15 | 100 | 89,8 | 0 | 97,48 | 0 |

**Tabelle 24: Messdaten und Ergebnisse bei 280 °C und 30 MPa in 50 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 38,06 | 0,26 | 0,19 | 0 | 0 | 0 | | |
| 48,3 | 8,50 | 2,82 | 26,33 | 77,7 | 6,7 | 68,7 | 8,68 | 88,4 |
| 74,9 | 6,09 | 4,74 | 27,10 | 84 | 11,8 | 70,7 | 14,04 | 84,2 |
| 90,2 | 5,34 | 5,58 | 27,10 | 86 | 14,0 | 70,7 | 16,27 | 82,2 |
| 185,6 | 2,03 | 11,09 | 24,49 | 94,7 | 28,5 | 63,8 | 30,08 | 67,4 |
| 279,1 | 0,75 | 14,75 | 21,31 | 98 | 38,1 | 55,5 | 38,84 | 56,6 |
| 371,6 | 0,23 | 17,63 | 18,13 | 99,4 | 45,7 | 47,1 | 45,93 | 47,4 |
| 552,2 | 0 | 21,67 | 12,79 | 100 | 56,3 | 33,1 | 56,27 | 33,1 |

**Tabelle 25: Messdaten und Ergebnisse bei 280 °C und 30 MPa in 1,17 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 35,3 | 0 | 0,57 | 0 | 0 | 0 | | |
| 48,9 | 0 | 7,06 | 25,83 | 100 | 20 | 72 | 20 | 72 |
| 73,5 | 0 | 10,79 | 22,56 | 100 | 31 | 62 | 31 | 62 |
| 107,4 | 0 | 13,25 | 20,07 | 100 | 38 | 55 | 38 | 55 |
| 202,3 | 0 | 17,42 | 15,48 | 100 | 49 | 42 | 49 | 42 |
| 281,6 | 0 | 20,60 | 12,26 | 100 | 58 | 33 | 58 | 33 |
| 468,7 | 0 | 24,34 | 8,19 | 100 | 69 | 22 | 69 | 22 |

**Tabelle 26: Messdaten und Ergebnisse bei 280 °C und 30 MPa in 5,88 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 38,3 | 0 | 0 | 0 | 0 | 0 | | |
| 53,5 | 0 | 14,9 | 23,1 | 100 | 39 | 60 | 39 | 60 |
| 77,0 | 0 | 19,4 | 18,4 | 100 | 51 | 48 | 51 | 48 |
| 103,3 | 0 | 22 | 15,4 | 100 | 57 | 40 | 57 | 40 |
| 190,0 | 0 | 26,9 | 9,6 | 100 | 70 | 25 | 70 | 25 |
| 303,3 | 0 | 30,4 | 5,1 | 100 | 79 | 13 | 79 | 13 |
| 545,6 | 0 | 33,2 | 1,7 | 100 | 87 | 4 | 87 | 4 |

**Tabelle 27: Messdaten und Ergebnisse bei 280 °C und 30 MPa in 14,7 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 33,85 | 0 | 4,45 | 0 | 0 | 0 | | |
| 50,6 | 0 | 13,21 | 24,49 | 100 | 39 | 59 | 39 | 59 |
| 102,7 | 0 | 22,19 | 15,58 | 100 | 66 | 33 | 66 | 33 |
| 200,8 | 0 | 29,30 | 8,27 | 100 | 87 | 11 | 87 | 11 |
| 293,9 | 0 | 31,61 | 5,72 | 100 | 93 | 4 | 93 | 4 |
| 500,7 | 0 | 32,77 | 4,45 | 100 | 97 | 0 | 97 | 0 |

**Tabelle 28: Messdaten und Ergebnisse bei 290 °C und 30 MPa in 5 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 37,61 1 | 0,19 | 0 | 0 | 0 | 0 | | |
| 51,2 | 7,97 | 3,33 | 25,83 | 79 | 8 | 69 | 11 | 87 |
| 73,7 | 6,02 | 5,19 | 26,08 | 84 | 13 | 69 | 16 | 83 |
| 101,6 | 4,44 | 6,86 | 25,44 | 88 | 18 | 68 | 20 | 77 |
| 188,7 | 1,58 | 12,31 | 22,07 | 96 | 32 | 59 | 34 | 61 |
| 284,4 | 0,53 | 16,03 | 18,45 | 99 | 42 | 49 | 43 | 50 |
| 384,2 | 0,15 | 17,95 | 14,82 | 100 | 47 | 39 | 47 | 40 |
| 572,6 | 0,00 | 19,88 | 9,22 | 100 | 52 | 25 | 52 | 25 |

**Tabelle 29: Messdaten und Ergebnisse bei 300 °C und 30 MPa in 5 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 37,2 | 0 | 0 | 0 | 0 | 0 | | |
| 72,9 | 11,75 | 4,24 | 19,63 | 68 | 11 | 53 | 17 | 77 |
| 100,1 | 8,87 | 5,72 | 20,61 | 76 | 15 | 55 | 20 | 73 |
| 190,8 | 3,24 | 10,69 | 19,82 | 91 | 29 | 53 | 31 | 58 |
| 278,9 | 1,23 | 13,58 | 16,89 | 97 | 37 | 45 | 38 | 47 |
| 454,8 | 0,07 | 15,49 | 11,16 | 100 | 42 | 30 | 42 | 30 |
| 617,9 | 0,00 | 15,00 | 8,05 | 100 | 40 | 22 | 40 | 22 |

**Tabelle 30: Messdaten und Ergebnisse bei 300 °C und 30 MPa in 10 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 37,35 | 0 | 0 | 0 | 0 | 0 | | |
| 75,6 | 9,30 | 5,29 | 21,83 | 75 | 14 | 58 | 19 | 78 |
| 99,1 | 6,78 | 7,31 | 21,77 | 82 | 20 | 58 | 24 | 71 |
| 190,6 | 2,09 | 12,85 | 19,15 | 94 | 34 | 51 | 36 | 54 |
| 458,1 | 0,00 | 15,61 | 9,82 | 100 | 42 | 26 | 42 | 26 |

**Tabelle 31: Messdaten und Ergebnisse bei 300 °C und 30 MPa in 50 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 38,65 | 0 | 0 | 0 | 0 | 0 | | |
| 48,5 | 7,93 | 3,50 | 26 | 79 | 9 | 67 | 11 | 85 |
| 72,0 | 5,70 | 5,59 | 26,6 | 85 | 14 | 69 | 17 | 81 |
| 97,2 | 3,97 | 7,87 | 26,3 | 90 | 20 | 68 | 23 | 76 |
| 176,5 | 1,15 | 13,28 | 21,9 | 97 | 34 | 57 | 35 | 58 |
| 275,2 | 0,36 | 17,09 | 16,9 | 99 | 44 | 44 | 45 | 44 |
| 444,2 | 0 | 19,42 | 11 | 100 | 50 | 28 | 50 | 28 |

**Tabelle 32: Messdaten und Ergebnisse bei 300 °C und 30 MPa in 1,17 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 31 | 0 | 0 | 0 | 0 | 0 | | |
| 50 | 0 9 | | 22 | 99 | 28 | 71 | 29 | 71 |
| 98 | 0 | 14 | 17 | 100 | 45 | 55 | 45 | 55 |
| 187 | 0 | 19 | 11 | 100 | 60 | 37 | 60 | 37 |
| 282 | 0 | 21 | 8 | 100 | 67 | 27 | 67 | 27 |
| 465 | 0 | 22 | 5 | 100 | 70 | 15 | 70 | 15 |

**Tabelle 33: Messdaten und Ergebnisse bei 300 °C und 30 MPa in 5,88 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 38 | 0 | 1 | 0 | 0 | 0 | | |
| 52 | 0 | 18 | 21 | 100 | 46 | 53 | 46 | 53 |
| 72 | 0 | 22 | 16 | 100 | 58 | 40 | 58 | 40 |
| 108 | 0 | 25 | 13 | 100 | 64 | 33 | 64 | 33 |
| 185 | 0 | 28 | 8 | 100 | 74 | 20 | 74 | 20 |
| 277 | 0 | 31 | 5 | 100 | 80 | 12 | 80 | 12 |
| 477 | 0 | 33 | 2 | 100 | 87 | 4 | 87 | 4 |

**Tabelle 34: Messdaten und Ergebnisse bei 300 °C und 30 MPa in 14,7 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 32 | 0 | 4 | 0 | 0 | 0 | | |
| 49 | 0 | 12 | 24 | 100 | 38 | 60 | 38 | 60 |
| 103 | 0 | 20 | 16 | 100 | 63 | 35 | 63 | 35 |
| 295 | 0 | 27 | 7 | 100 | 83 | 9 | 83 | 9 |
| 511 | 0 | 29 | 5 | 100 | 89 | 1 | 89 | 1 |

**Tabelle 35: Messdaten und Ergebnisse bei 330 °C und 30 MPa in 5 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 35 | 0 | 0 | 0 | 0 | 0 | | |
| 54 | 8 | 4 | 19 | 76 | 12 | 55 | 16 | 73 |
| 101 | 4 | 5 | 18 | 89 | 15 | 53 | 16 | 60 |
| 195 | 0 | 5 | 13 | 99 | 13 | 36 | 14 | 37 |
| 284 | 0 | 4 | 8 | 100 | 11 | 23 | 11 | 23 |
| 465 | 0 | 2 | 3 | 100 | 6 | 9 | 6 | 9 |

**Tabelle 36: Messdaten und Ergebnisse bei 330 °C und 30 MPa in 50 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 36 | 0 | 0 | 0 | 0 | 0 | | |
| 47 | 10 | 4 | 19 | 71 | 12 | 53 | 17 | 74 |
| 102 | 3 | 7 | 17 | 92 | 18 | 48 | 20 | 52 |
| 195 | 0 | 5 | 11 | 99 | 14 | 30 | 14 | 30 |
| 292 | 0 | 4 | 6 | 100 | 10 | 17 | 10 | 17 |
| 469 | 0 | 2 | 2 | 100 | 5 | 6 | 5 | 6 |

**Tabelle 37: Messdaten und Ergebnisse bei 330 °C und 30 MPa in 1,17 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 33 | 0 | 0 | 0 | 0 | 0 | | |
| 49 | 0 | 11 | 21 | 99 | 32 | 62 | 32 | 63 |
| 99 | 0 | 14 | 14 | 100 | 41 | 43 | 41 | 43 |
| 196 | 0 | 13 | 8 | 100 | 39 | 23 | 39 | 23 |
| 292 | 0 | 11 | 3 | 100 | 32 | 10 | 32 | 10 |
| 463 | 0 | 7 | 1 | 100 | 20 | 4 | 20 | 4 |

**Tabelle 38: Messdaten und Ergebnisse bei 330 °C und 30 MPa in 5,88 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 37 | 0 | 0 | 0 | 0 | 0 | | |
| 46 | 0 | 16 | 19 | 100 | 44 | 53 | 45 | 53 |
| 74 | 0 | 19 | 15 | 100 | 51 | 42 | 51 | 42 |
| 101 | 0 | 21 | 12 | 100 | 56 | 32 | 56 | 32 |
| 161 | 0 | 22 | 8 | 100 | 60 | 22 | 60 | 22 |
| 294 | 0 | 20 | 4 | 100 | 54 | 10 | 54 | 10 |
| 487 | 0 | 15 | 1 | 100 | 40 | 2 | 40 | 2 |

**Tabelle 39: Messdaten und Ergebnisse bei 330 °C und 30 MPa in 14,7 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 27 | 0 | 7 | 0 | 0 | 0 | | |
| 48 | 0 | 11 | 20 | 100 | 41 | 49 | 41 | 49 |
| 105 | 0 | 15 | 16 | 100 | 55 | 34 | 55 | 34 |
| 298 | 0 | 15 | 10 | 100 | 55 | 11 | 55 | 11 |
| 490 | 0 | 12 | 8 | 100 | 46 | 1 | 46 | 1 |

**Tabelle 40: Messdaten und Ergebnisse bei 350 °C und 30 MPa in 5 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 33 | 0 | 0 | 0 | 0 | 0 | | |
| 48 | 7 | 3 | 17 | 82 | 9 | 52 | 11 | 65 |
| 100 | 1 | 2 | 13 | 96 | 7 | 38 | 8 | 39 |
| 187 | 0 | 1 | 5 | 100 | 4 | 16 | 4 | 16 |
| 278 | 0 | 1 | 2 | 100 | 2 | 6 | 2 | 6 |
| 377 | 0 | 0 | 1 | 100 | 1 | 2 | 1 | 2 |
| 469 | 0 | 0 | 0 | 100 | 0 | 0 | 0 | 0 |

**Tabelle 41: Messdaten und Ergebnisse bei 380 °C und 30 MPa in 5 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 28 | 0 | 0 | 0 | 0 | 0 | | |
| 31 | 5 | 0,8 | 14 | 81 | 3 | 49 | 4 | 61 |
| 48 | 2 | 0,8 | 11 | 94 | 3 | 40 | 3 | 43 |
| 97 | 0 | 0,3 | 4 | 100 | 1 | 14 | 1 | 14 |
| 194 | 0 | 0 | 0 | 100 | 0 | 1 | 0 | 1 |
| 287 | 0 | 0 | 0 | 100 | 0 | 0 | 0 | 0 |

**Tabelle 42: Messdaten und Ergebnisse bei 400 °C und 30 MPa in 5 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 20,0 | 0,0 | 0,0 | 0 | 0 | 0 | | |
| 20 | 3,4 | 0,8 | 7,5 | 83 | 4 | 38 | 5 | 46 |
| 42 | 0,2 | 0,1 | 1,8 | 99 | 1 | 9 | 1 | 9 |
| 75 | 0,0 | 0,0 | 0,3 | 100 | 0 | 2 | 0 | 2 |
| 156 | 0,0 | 0,0 | 0,0 | 100 | 0 | 0 | 0 | 0 |
| 259 | 0,0 | 0,0 | 0,0 | 100 | 0 | 0 | 0 | 0 |

### Beispiel 4: Hydrolyse von 5, 10 und 15 Gew.-% Nikotinonitril in konz. Ammoniak (14,7 M, 25%)

**Tabelle 43: Messdaten und Ergebnisse bei 250 °C und 30 MPa in 14,7 M Ammoniak und 5 Gew.-% Nikotinonitril**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 366 | 0 | 28 | 0 | 0 | 0 | | |
| 117 | 26 | 112 | 257 | 93 | 31 | 62 | 33 | 67 |
| 238 | 0 | 200 | 193 | 100 | 55 | 45 | 55 | 45 |
| 590 | 0 | 294 | 85 | 100 | 80 | 15 | 80 | 15 |
| 801 | 0 | 314 | 61 | 100 | 86 | 9 | 86 | 9 |

**Tabelle 44: Messdaten und Ergebnisse bei 250 °C und 30 MPa in 14,7 M Ammoniak und 10 Gew.-% Nikotinonitril**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 791 | 0 | 57 | 0 | 0 | 0 | | |
| 47 | 211 | 57 | 576 | 73 | 7 | 66 | 10 | 89 |
| 117 | 72 | 177 | 596 | 91 | 22 | 68 | 25 | 75 |
| 223 | 20 | 294 | 513 | 97 | 37 | 58 | 38 | 59 |
| 576 | 0 | 526 | 311 | 100 | 67 | 32 | 67 | 32 |
| 817 | 0 | 586 | 225 | 100 | 74 | 21 | 74 | 21 |

**Tabelle 45: Messdaten und Ergebnisse bei 250 °C und 30 MPa in 14,7 M Ammoniak und 15 Gew.-% Nikotinonitril**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 1109 | 0 | 75 | 0 | 0 | 0 | | |
| 123 | 199 | 97 | 887 | 82 | 9 | 73 | 11 | 89 |
| 219 | 76 | 240 | 867 | 93 | 22 | 71 | 23 | 77 |
| 574 | 0 | 610 | 571 | 100 | 55 | 45 | 55 | 45 |
| 852 | 0 | 731 | 451 | 100 | 66 | 34 | 66 | 34 |

**Tabelle 46: Messdaten und Ergebnisse bei 280 °C und 30 MPa in 14,7 M Ammoniak und 5 Gew.-% Nikotinonitril**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 366 | 0 | 8 | 0 | 0 | 0 | | |
| 52 | 35 | 63 | 275 | 90 | 17 | 73 | 19 | 79 |
| 106 | 15 | 136 | 214 | 96 | 37 | 56 | 39 | 66 |
| 292 | 0 | 232 | 141 | 100 | 63 | 36 | 63 | 39 |
| 512 | 0 | 269 | 105 | 100 | 73 | 26 | 73 | 30 |
| 724 | 0 | 275 | 94 | 100 | 75 | 24 | 75 | 26 |

**Tabelle 47: Messdaten und Ergebnisse bei 280 °C und 30 MPa in 14,7 M Ammoniak und 10 Gew.-% Nikotinonitril**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 1038 | 0 | 81 | 0 | 0 | 0 | | |
| 51 | 232 | 63 | 824 | 78 | 6 | 72 | 8 | 92 |
| 106 | 119 | 158 | 842 | 89 | 15 | 73 | 17 | 83 |
| 312 | 0 | 386 | 659 | 100 | 37 | 56 | 37 | 56 |
| 505 | 0 | 542 | 554 | 100 | 52 | 46 | 52 | 46 |
| 699 | 0 | 599 | 493 | 100 | 58 | 40 | 58 | 40 |

**Tabelle 48: Messdaten und Ergebnisse bei 300 °C und 30 MPa in 14,7 M Ammoniak und 5 Gew.-% Nikotinonitril**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 352 | 0 | 13 | 0 | 0 | 0 | | |
| 53 | 77 | 49 | 239 | 78 | 14 | 64 | 18 | 82 |
| 106 | 39 | 100 | 220 | 89 | 28 | 59 | 32 | 66 |
| 311 | 0 | 171 | 182 | 100 | 49 | 48 | 49 | 48 |
| 522 | 0 | 191 | 160 | 100 | 54 | 42 | 54 | 42 |
| 765 | 0 | 189 | 149 | 100 | 54 | 38 | 54 | 38 |

**Tabelle 49: Messdaten und Ergebnisse bei 300 °C und 30 MPa in 14,7 M Ammoniak und 10 Gew.-% Nikotinonitril**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 692 | 0 | 15 | 0 | 0 | 0 | n.b. | n.b. |
| 50 | 149 | 80 | 482 | 78 | 12 | 67 | 15 | 86 |
| 104 | 67 | 147 | 465 | 90 | 21 | 65 | 24 | 72 |
| 204 | 0 | 234 | 424 | 100 | 34 | 59 | 34 | 59 |
| 317 | 0 | 283 | 374 | 100 | 41 | 52 | 41 | 52 |
| 515 | 0 | 321 | 321 | 100 | 46 | 44 | 46 | 44 |
| 781 | 0 | 333 | 304 | 100 | 48 | 42 | 48 | 42 |

**Tabelle 50: Messdaten und Ergebnisse bei 300 °C und 30 MPa in 14,7 M Ammoniak und 15 Gew.-% Nikotinonitril**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 1041 | 0 | 35 | 0 | 0 | 0 | n.b. | n.b. |
| 52 | 157 | 77 | 797 | 85 | 7 | 73 | 9 | 86 |
| 107 | 50 | 146 | 781 | 95 | 14 | 72 | 15 | 75 |
| 265 | 0 | 301 | 681 | 100 | 29 | 62 | 29 | 62 |
| 492 | 0 | 407 | 564 | 100 | 39 | 51 | 39 | 51 |
| 784 | 0 | 436 | 499 | 100 | 42 | 45 | 42 | 45 |

**Tabelle 51: Messdaten und Ergebnisse bei 330 °C und 30 MPa in 14,7 M Ammoniak und 5 Gew.-% Nikotinonitril**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 287 | 0 | 36 | 0 | 0 | 0 | n.b. | n.b. |
| 52 | 56 | 39 | 189 | 80 | 14 | 53 | 17 | 66 |
| 111 | 16 | 81 | 193 | 94 | 28 | 54 | 30 | 58 |
| 285 | 0 | 88 | 169 | 100 | 31 | 46 | 31 | 46 |
| 607 | 0 | 60 | 126 | 100 | 21 | 31 | 21 | 31 |
| 774 | 0 | 44 | 108 | 100 | 15 | 25 | 15 | 25 |

**Tabelle 52: Messdaten und Ergebnisse bei 330 °C und 30 MPa in 14,7 M Ammoniak und 15 Gew.-% Nikotinonitril**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 635 | 0 | 40 | 0 | 0 | 0 | n.b. | n.b. |
| 52 | 165 | 97 | 397 | 74 | 15 | 56 | 21 | 76 |
| 110 | 87 | 142 | 406 | 86 | 22 | 58 | 26 | 67 |
| 213 | 36 | 160 | 378 | 94 | 25 | 53 | 27 | 56 |
| 307 | 0 | 148 | 344 | 100 | 23 | 48 | 23 | 48 |
| 509 | 0 | 118 | 262 | 100 | 19 | 35 | 19 | 35 |

**Tabelle 53: Messdaten und Ergebnisse bei 330 °C und 30 MPa in 14,7 M Ammoniak 15% Nikotinonitril**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 939 | 0 | 47 | 0 | 0 | 0 | | |
| 101 | 53 | 198 | 618 | 94 | 21 | 61 | 22 | 65 |
| 279 | 0 | 192 | 556 | 100 | 20 | 54 | 20 | 54 |
| 469 | 0 | 142 | 438 | 100 | 15 | 42 | 15 | 42 |
| 676 | 0 | 79 | 330 | 100 | 8 | 30 | 8 | 30 |

## Patentansprüche

1. Ein Verfahren zur Hydrolyse von Verbindungen der Formeln 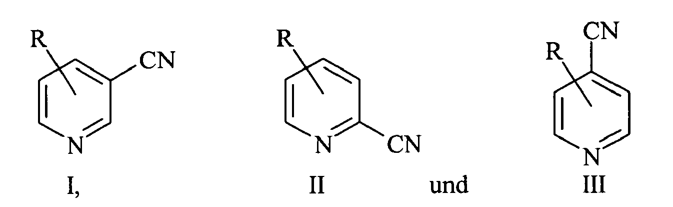 worin R Wasserstoff oder C₁₋₂₀-Alkyl ist,
zu den entsprechenden Amiden und/oder Säuren in wässrigen Fluiden, **dadurch gekennzeichnet, dass** die Reaktion bei einer Temperatur von mindestens 100 °C und einem Druck von mindestens 20 kPa, gegebenenfalls in Gegenwart eines sauren oder basischen Katalysators, durchgeführt wird.

2. Verfahren nach Anspruch 1, **dadurch gekennzeichnet, dass** die Reaktion kontinuierlich in einem Strömungsreaktor durchgeführt wird.

3. Verfahren nach Anspruch 2, **dadurch gekennzeichnet, dass** der Strömungsreaktor mindestens eine Reaktionszone aufweist.

4. Verfahren nach Anspruch 2 oder 3, **dadurch gekennzeichnet, dass** die Verweildauer in der Reaktionszone zwischen 0,1 und 2000 Sekunden beträgt.

5. Verfahren nach einem der Ansprüche 1 bis 4, **dadurch gekennzeichnet, dass** das wässrige Fluid zu Beginn der Reaktion die Verbindung der Formel I in einer Konzentration von 0,05 bis 30 Gew.-% enthält.

6. Verfahren nach einem der Ansprüche 1 bis 5, **dadurch gekennzeichnet, dass** es bei einem Druck von 20 bis 60 kPa durchgeführt wird.

7. Verfahren nach einem der Ansprüche 1 bis 6, **dadurch gekennzeichnet, dass** der Katalysator eine organische oder anorganische Säure ist.

8. Verfahren nach einem der Ansprüche 1 bis 6, **dadurch gekennzeichnet, dass** der Katalysator eine organische oder anorganische Base ist.

9. Verfahren nach Anspruch 8, **dadurch gekennzeichnet, dass** die anorganische Base ausgewählt ist aus der Gruppe bestehend aus Hydroxiden und Oxiden von Metallen der ersten und zweiten Hauptgruppe, Ammoniak, Triethylamin, Trimethylamin und Mischungen davon.
